Europäisches Patentamt

**European Patent Office**

**Office européen des brevets**

(11) Veröffentlichungsnummer : **0 027 890**
**B1**

(12) **EUROPÄISCHE PATENTSCHRIFT**

(45) Veröffentlichungstag der Patentschrift :
22.06.83

(21) Anmeldenummer : 80105655.7

(22) Anmeldetag : 20.09.80

(51) Int. Cl.³ : **C 07 C 49/92**, C 07 C 45/77,
C 07 F 15/06

(54) Verfahren zur Herstellung von Cobalt(II)-acetylacetonat.

(30) Priorität : 19.10.79 NL 7907739

(43) Veröffentlichungstag der Anmeldung :
06.05.81 Patentblatt 81/18

(45) Bekanntmachung des Hinweises auf die Patenterteilung : 22.06.83 Patentblatt 83/25

(84) Benannte Vertragsstaaten :
AT BE CH DE FR GB IT LI LU NL SE

(56) Entgegenhaltungen :
DE C 1 039 056

(73) Patentinhaber : Chemische Fabriek Zaltbommel B.V.
Postbus 52 Koxkampseweg 14-20
Zaltbommel (NL)

(72) Erfinder : van der Maas, Hendrikus J. H.
De Boogerd 25
NL-5305 CK Zuilichem, Gemeinde Brakel (NL)

(74) Vertreter : Siecke, Wolf-Friedrich, Dr.
c/o DYNAMIT NOBEL AKTIENGESELLSCHAFT HA
Patente/Patentabteilung Postfach 1209 Kölner
Strasse
D-5210 Troisdorf/Bez. Köln (DE)

Jouve, 18, rue St-Denis, 75001 Paris, France

Verfahren zur Herstellung von Cobalt(II)-acetylacetonat

Gegenstand der vorliegenden Erfindung ist ein Verfahren zur Herstellung von Cobalt(II)-acetylacetonat. Bei diesem Verfahren wird in an sich bekannter Weise eine Cobaltverbindung mit Acetylaceton bei erhöhter Temperatur umgesetzt. Die Umsetzung findet jedoch in bestimmten organischen Lösungsmitteln statt, wobei die Reaktionstemperatur 100 °C nicht zu übersteigen braucht.

Es ist bekannt, Cobalt(II)-acetylacetonat durch Umsetzung von Cobalt(II)-verbindungen mit Acetylaceton herzustellen. Als Cobaltverbindungen eignen sich dabei Salze anorganischer Säuren, wie z. B. das Chlorid, Sulfat, Nitrat, Carbonat oder auch das Hydroxid ; auch Cobaltoxid ist einsetzbar. Die Umsetzung wird im wässrigen Medium durchgeführt ; dabei muß mit Ammoniak oder einer anderen schwachen Base neutralisiert werden.

Bei dieser Arbeitsweise fällt das kristallwasserhaltige Produkt an, das zwei Mol Kristallwasser gebunden enthält. Die Entfernung des Kristallwassers ist aufwendig, weil sie bei vermindertem Druck (zwischen 20 und 2 mbar) und Temperaturen bis zu 90 °C durchgeführt werden muß. Nachteilig wirkt sich bei dieser Arbeitsweise noch zusätzlich aus, daß dabei das trockene Produkt sehr feinkörnig anfällt und leicht zerstäubt wird.

Es ist auch schon durch die DE-PS 10 39 056 bekannt, wasserfreie Metallacetylacetonate in der Weise herzustellen, daß man Metallverbindungen mit wasserfreiem Ammoniumacetylacetonat in Gegenwart eines wasserfreien organischen Lösungsmittels umsetzt. Bei dieser Arbeitsweise entstehen zwar ebenfalls die wasserfreien Metallacetylacetonate ; jedoch befinden sich diese Verbindungen in Lösung ; ein einfaches Abdampfen des Lösungsmittels, wie es in der Patentschrift vorgeschlagen wird, führt nicht zu reinen Acetylacetonaten, weil in dem Lösungsmittel immer noch geringe Mengen an Ammoniumsalzen gelöst sind. Weiterhin ist bei dieser Arbeitsweise von Nachteil, daß in einem gesonderten Arbeitsgang das als Ausgangsprodukt einzusetzende Ammoniumacetylacetonat in wasserfreier Form hergestellt werden muß.

Es bestand deshalb die Aufgabe, wasserfreies Cobalt(II)-acetylacetonat so herzustellen, daß dabei nicht gleichzeitig auch ein Ammoniumsalz anfällt, von dem das anfallende Rohprodukt abgetrennt und anschließend gereinigt werden muß. Weiterhin bestand die Aufgabe, ein Verfahren zu finden, bei dem ein Produkt anfällt, das sich leicht zu einem wasserfreien Produkt trocknen läßt.

In Erfüllung dieser Aufgabe wurde nun ein Verfahren zur Herstellung von Cobalt(II)-acetylacetonat durch Umsetzung von Cobalt(II)-verbindungen mit Acetylaceton gefunden, das dadurch gekennzeichnet ist, daß man die Umsetzung in einem organischen Lösungsmittel, das mit Wasser nicht mischbar ist und ein azeotropes Gemisch mit Wasser bildet, im Bereich der Siedetemperatur des azeotropen Gemischs durchführt.

Eine bevorzugte Arbeitsweise des neuen Verfahrens besteht darin, das bei der Reaktion sich bildende azeotrope Gemisch aus dem organischen Lösungsmittel und Wasser kontinuierlich abzudestillieren. Das Destillat kann dabei dann in die organische und die wässrige Phase aufgetrennt werden ; die organische Phase wird dann dem Reaktionsgemisch wieder hinzugeführt. Diese Arbeitsweise ermöglicht zusätzlich noch den Fortgang der Reaktion zu kontrollieren, indem die Menge des abdestillierten Wassers bestimmt wird. Das Ende der Reaktion ist dann daran erkennbar, daß kein Wasser als azeotropes Gemisch mehr abdestilliert.

Es ist jedoch auch möglich, das azeotrope Gemisch aus dem Lösungsmittel und Wasser nicht kontinuierlich abzudestillieren ; man erhitzt dann das Reaktionsgemisch unter Rückfluß auf Temperaturen bis zur Siedetemperatur dieses azeotropen Gemisches. Die Umsetzung läuft auch ab, wenn das Lösungsmittelgemisch noch nicht siedet, so daß das erfindungsgemäße Verfahren allgemein bei Temperaturen zwischen 60 °C und 100 °C durchführbar ist. Es ist bei einer solchen Arbeitsweise jedoch von Vorteil, gegen Ende der Reaktion das azeotrope Gemisch abzudestillieren.

Die Menge des im Reaktionsraum vorhandenen organischen Lösungsmittels soll mindestens so groß sein, daß das erhaltene Cobalt(II)-acetylacetonat darin bei der Reaktionstemperatur gelöst vorliegt. Diese Menge richtet sich nach der Art des gewählten Lösungsmittels. Weiterhin soll sie mindestens so groß sein, daß beim Abkühlen eine gut filtrierbare Suspension des gewünschten Salzes in dem Lösungsmittel vorliegt.

Sobald die Reaktion beendet ist, läßt man das Reaktionsgemisch abkühlen ; dabei fällt das Cobalt(II)-acetylacetonat aus. Im Gegensatz zu der Arbeitsweise im wässrigen Medium fällt bei der erfindungsgemäßen Verfahrensweise ein praktisch kristallwasserfreies Produkt aus. Nach dem Abfiltrieren läßt sich dieses auf einfache, an sich bekannte Weise trocknen ; dabei soll das Produkt nach Möglichkeit nicht über 90 °C erwärmt werden, weil es sich dann zu zersetzen beginnt. Vorzugsweise wird das Produkt deshalb unter Vakuum getrocknet, wobei ein Druck von 20 bis 100 mbar, vorzugsweise 30 bis 60 mbar, vollkommen ausreicht.

Bei höheren Drücken muß entsprechend länger getrocknet werden.

Die Ausbeuten an getrocknetem Endprodukt liegen im allgemeinen zwischen 90 und 96 Gew.-%, bezogen auf eingesetzte Cobaltverbindung. Sie sind damit erheblich höher als bei den bekannten Verfahren, bei denen Ausbeuten zwischen 60 und 75 % erhalten werden.

Als Lösungsmittel, die sich erfindungsgemäß einsetzen lassen, eignen sich sowohl aromatische als auch aliphatische oder cycloaliphatische Lösungsmittel. Voraussetzung ist nur, daß sie mit Wasser praktisch nicht mischbar sind und mit Wasser ein azeotropes Gemisch bilden, dessen Siedepunkt unter 100 °C liegt. Beispiele für aromatische Kohlenwasserstoffe, die diese Bedingungen erfüllen, sind Benzol, Anisol, die Xylole, Chlorbenzol. Zu den einsetzbaren aliphatischen Lösungsmitteln zählen aliphatische gesättigte Kohlenwasserstoffe, Chlorkohlenwasserstoffe, Ketone und Alkylester niedriger Carbonsäuren. Als Beispiele seien genannt : Hexan, Heptan, Nonan, Cyclohexan, Dichloräthan, Trichloräthan, Cyclohexanon, Cyclopentanon, die Methyl- bis Butylester der $C_1$ bis $C_4$-Carbonsäuren wie z. B. Ameisensäurebutylester, Essigsäurepropylester, Propansäureäthylester und Allylacetat. Die bevorzugten Lösungsmittel sind Benzol und n-Heptan.

Die bevorzugte Cobaltverbindung ist Cobalt(II)-hydroxid. Die Reaktion kann jedoch auch mit basischem Cobalt(II)-carbonat durchgeführt werden. Die Reaktionspartner werden im allgemeinen in stöchiometrischen Verhältnissen zur Reaktion gebracht, da die Ausbeuten des erfindungsgemäßen Verfahrens nahezu quantitativ sind. Es empfiehlt sich jedoch, das Acetylaceton in geringem Überschuß, der bis zu 10 % der stöchiometrisch notwendigen Menge betragen kann, einzusetzen. Dieser Überschuß kann nach dem Abiltrieren des Cobalt(II)-acetylacetonat zusammen mit der zurückgewonnenen Mutterlauge einem neuen Ansatz zugeführt werden. Eine Aufarbeitung der Mutterlauge ist dabei nicht notwendig.

Cobalt(II)-acetylacetonat dient als Katalysator für Polymerisation und Copolymerisationen, z. B. von Methacrylsäureestern oder von Butadien zu stereospezifischen Produkten, als Katalysator für Flüssigphasenoxidation oder für Hydrierungen. Weiterhin wird es als Beschichtungsmittel für Glasoberflächen oder als Antioxidans in Schmiermitteln eingesetzt.

Beispiel 1

In einem mit einer Destillationsapparatur versehenen Reaktionskolben werden 4 600 ml n-Heptan vorgelegt und darin 1 068,1 g (11,5 Mol) Cobalthydroxid suspendiert. Dieses Gemisch wird auf eine Temperatur von 90 bis 94 °C aufgewärmt, dabei tritt leichter Rückfluß ein. Innerhalb von vier Stunden werden zu diesem Gemisch 2 417,5 g Acetylaceton hinzugefügt. Die Temperatur des Reaktionsgefäßes wurde während des Züfügens auf 90 bis 94 °C gehalten. Im Laufe der Reaktion nahm der Rückfluß zu. Das übergehende Azeotrop wurde aufgefangen und in einem Scheidetrichter das Wasser abgeschieden, während das überstehende Heptan wiederum dem Reaktionsgemisch hinzugefügt wurde.

Im Laufe von 8 Stunden wurden auf diese Weise 405 ml Wasser abgeschieden. Die entspricht 98 % der theoretisch abspaltbaren Menge von 414 ml.

Daraufhin wurde das Reaktionsgemisch auf 15 °C abgekühlt. Dabei fallen tief dunkelrot gefärbte Kristalle von Cobalt(II)-acetylacetonat aus ; diese werden abfiltriert und mit insgesamt 1 000 ml Heptan ausgewaschen.

Anschließend wird das Lösungsmittel scharf abgesaugt.

Nach dem Trocknen des auf diese Weise erhaltenen Produktes in einem Ofen bei einer Temperatur von 40 °C und einem Druck von 40 mbar erhält man insgesamt 2 856,8 g des gewünschten Cobalt(II)-acetylacetonats. Dies entspricht einer Ausbeute von 96,6 %. Der Cobaltgehalt lag zwischen 22,6 und 22,7 % ; der Wassergehalt lag unter 1 %.

Beispiel 2

In dem Reaktionsgefäß des Beispiels 1 wurden die Mutterlauge des Beispiels 1 sowie so viel des Waschheptans dieses Beispiels vorgelegt, daß die gesamte Flüssigkeitsmenge 4 600 ml betrug. Darin wurden wiederum 1 068,8 g Cobalthydroxid vorgelegt. Die weitere Verarbeitung erfolgte in gleicher Weise, wie in Beispiel 1 beschrieben.

Es wurden insgesamt 2 862,4 g Cobalt(II)-acetylacetonat erhalten ; dies entspricht einer Ausbeute von 96,7 %. Der Cobaltgehalt lag zwischen 22,6 und 22,7 % ; der Wassergehalt war niedriger als 0,5 %.

Beispiel 3

In einer Apparatur entsprechend derjenigen des Beispiels 1 wurden 128 g basisches Cobaltcarbonat in 500 ml n-Heptan aufgerührt. Unter Rühren wurden insgesamt 212 g Acetylaceton hinzugefügt. Dabei war sofort eine Gasentwicklung erkennbar und die Innentemperatur stieg auf 45 °C. Weiterbin war eine Veränderung der Farbe und der Kristallform des Festprodukts wahrnehmbar.

Das Reaktionsgemisch wurde daraufhin während 5 Stunden bei Siedetemperatur gehalten, wobei insgesamt 54 ml Wasser aus dem übergehenden azeotropen Gemisch aufgefangen wurden. Im Laufe dieser Zeit färbte sich das anfangs rosa aussehende Cobaltacetylacetonat nach tiefviolett.

Anschließend wurde das Reaktionsgemisch auf 50 °C abgekühlt und der Feststoff abfiltriert und mit 75 ml Heptan ausgewaschen. Nach dem Trocknen wurden 253,1 g Cobalt(II)-acetylacetonat mit einem Metallgehalt zwischen 22,9 und 23,0 % und einem Wassergehalt von maximal 0,5 % erhalten. Die Ausbeute lag demzufolge bei 98,5 %.

Beispiel 4

Es wurden 128 g basisches Cobaltcarbonat in der Mutterlauge und dem Waschwasser des Beispiels 3 suspendiert. Die Lösungsmittelmenge betrug insgesamt 550 ml. In diese Suspension wurden unter Rühren 212 g Acetylaceton hinzugefügt. Dabei stieg die Innentemperatur auf 43 °C an und es war eine deutliche Gasentwicklung zu erkennen.

Das Reaktionsgemisch wurde anschließend während 6 Stunden beim Sieden gehalten. Dabei gingen insgesamt 53,6 ml Wasser in Form des Azeotrops mit Heptan über.

Die weitere Aufarbeitung erfolgt gemäß Beispiel 3. Insgesamt wurden 254,3 g Cobalt(II)-acetylacetonat mit einem Metallgehalt von 22,8 bis 22,9 % erhalten. Der Wassergehalt lag unter 0,5 %.

## Ansprüche

1. Verfahren zur Herstellung von wasserfreiem Cobalt(II)-acetylacetonat durch Umsetzung von Cobalt(II)-Verbindungen mit Acetylaceton bei erhöhter Temperatur, dadurch gekennzeichnet, daß man die Umsetzung in einem organischen Lösungsmittel, das mit Wasser nicht mischbar ist und mit Wasser ein bei Temperaturen bis zu 100 °C siedendes azeotropes Gemisch bildet, im Bereich der Siedetemperatur dieses azeotropen Gemisches durchführt.

2. Verfahren gemäß Anspruch 1, dadurch gekennzeichnet, daß man das bei der Reaktion entstehende azeotrope Gemisch aus Lösungsmittel und Wasser im Lauf seiner Entstehung abdestilliert.

## Claims

1. Process for the preparation of water-free cobalt(II)-acetylacetonate by reaction of cobalt(II)-compounds with acetylacetone at elevated temperature, characterised in that the reaction is carried out in an organic solvent which is not miscible with water and forms with water an azeotropic mixture boiling at temperatures of up to 100 °C, in the region of the boiling temperature of this azeotrope.

2. Process according to claim 1, characterised in that the azeotropic mixture originating during the reaction is distilled off from solvent and water during the course of its formation.

## Revendications

1. Procédé pour la préparation d'acétylacéto-nate de cobalt(II) anhydre par réaction de composés de cobalt(II) avec de l'acétylacétone à température élevée, caractérisé en ce qu'on effectue la réaction dans un solvant organique non miscible avec l'eau et formant avec l'eau un mélange azéotropique bouillant à des températures allant jusqu'à 100 °C, dans le domaine d'ébullition dudit mélange azéotropique.

2. Procédé selon la revendication 1, caractérisé en ce qu'on sépare par distillation, au cours de sa formation, le mélange azéotropique de solvant et d'eau se formant pendant la réaction.